(19) Europäisches Patentamt
European Patent Office
Office européen des brevets

(11) Publication number: **0 408 330 A2**

# (12) EUROPEAN PATENT APPLICATION

(21) Application number: **90307565.3**

(22) Date of filing: **11.07.90**

(51) Int. Cl.5: **C07J 13/00**, C07J 51/00, A61K 31/575

(30) Priority: **13.07.89 IE 2257/89**

(43) Date of publication of application: **16.01.91 Bulletin 91/03**

(84) Designated Contracting States: **AT BE CH DE DK ES FR GB GR IT LI LU NL SE**

(71) Applicant: **ELAN CORPORATION P.L.C.**
**Monksland Industrial Estate**
**Athlone County Westmeath(IE)**

(72) Inventor: **Masterson, Joseph G.**
**Porterstown**
**Clonsilla, Dublin 15(IE)**

(74) Representative: **Goldin, Douglas Michael et al**
**J.A. KEMP & CO. 14, South Square Gray's Inninn**
**London WC1R 5EU(GB)**

## (54) Anti-infective agents, process for their preparation and their use in therapy.

(57) Transition metal complexes or salts of fusidic acid, especially the silver and zinc salts thereof, are useful as anti-infective agents. The salts are particularly suitable for topical application.

EP 0 408 330 A2

This invention relates to broad spectrum anti-infective agents with particular application in the topical treatment of burns, lesions and open wounds.

Successful treatment of burns, lesion sites and open wounds can be difficult due to infection. When an epithelial surface is broken the tissues may be invaded by foreign material such as bacteria, dirt and fragments of clothing, which may give rise to serious local or general complications from infections. Infection caused by virulent microorganisms nourished by dead tissue and organic foreign material in a wound may take several forms including infections caused by such organisms as Streptococcus and Staphylococcus , as well as, Gram-negative bacteria and Clostridia . Bacteria can acquire resistance to antibiotics and anti-infective agents generally with time. Accordingly, new effective anti-infective agents are constantly sought as bacteria and other microorganisms acquire resistance to known agents.

Silver is known to possess antibacterial properties and is used topically as an antibacterial treatment for burns either as a metal or as silver salts. Dilute solutions (approximately 0.5%) of silver nitrate have been used as a 'wet-soak' to cover burn wounds. The conventional treatment involves complete wrapping of a patient's burn wounds with thick gauze bandages saturated with silver nitrate. Such treatment retards bacterial growth and allows epithelization in deep second degree burns. The bacteriostatic effect is only on the surface of the burn wound and does not penetrate. Only the use of dilute solutions of silver nitrate is possible because concentrated silver nitrate destroys live tissue in addition to killing bacteria.

Silver sulphadiazine - the silver salt of $N^1$-(pyrimidin-2-yl) sulphanilamide is used, in conjunction with debridement, for the prevention and treatment of infection, particularly by Pseudomonas aeruginosa , in severe burns. Treatment is generally continued until healing is progressing satisfactorily or the burn site is ready for skin grafting. Silver sulphadiazine has also been used in other skin conditions, such as leg ulcers, where infection may prevent healing.

It is also known that zinc possesses antibacterial, antiviral and antifungal activity and is often incorporated into liquids, powders, ointments or aerosols as various salts and applied topically for the treatment of, e.g., conjunctivitis, acne, impetigo and tinea pedia (athletes foot).

Fusidic acid and various more or less water soluble salts with atoxic inorganic or organic bases are effective against a number of pathogenic microorganisms. Fusidic acid has been used systemically and topically in the treatment of bacterial infection, especially Staphylococcal infections. It has only weak activity against gram negative bacilli and fungi.

Neither the silver salt, the zinc salt nor other transition metal salts of fusidic acid have been described in the literature. Known salts of fusidic acid include the sodium, potassium, calcium, magnesium, ammonium, triethylamine and other amine salts.

It is an object of the present invention to provide broad spectrum anti-infective agents with particular application in the topical treatment of infections and which promote healing of burns, lesions and open wounds.

We have found that transition metal complexes or salts, especially the silver and zinc salts, of fusidic acid, more particularly the silver salt, meet this objective.

Accordingly, the invention provides in one aspect a transition metal complex or salt of fusidic acid. The salts have the general formula:

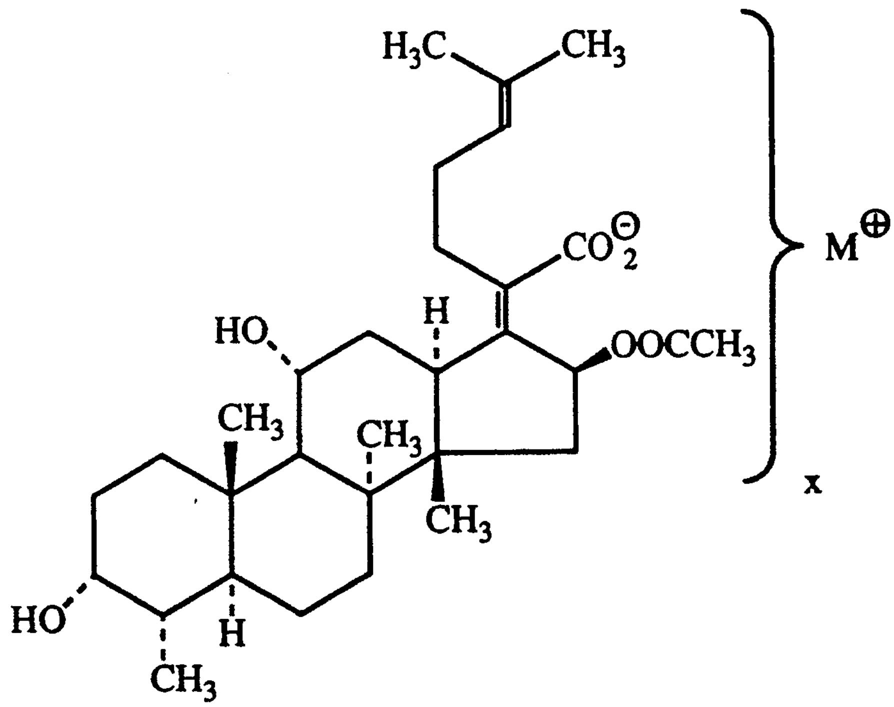

wherein x is the number of molecules necessary to satisfy the valency of M and M is a transition metal.

Especially suitable salts are the silver and zinc salts of fusidic acid.

Thus the invention provides silver fusidate which is insoluble in water and hexane and soluble in methanol, ethyl acetate and acetone; melts at 200-202°C; has a molecular weight of 623.55; has an empirical formula $C_{31}$ $H_{47}$ Ag $O_6$; and on thin layer chromatography on silica gel with 85:15 v/v chloroform-methanol with uv visualisation shows one major spot at $R_f$ 0.65.

Silver fusidate has the structural formula:

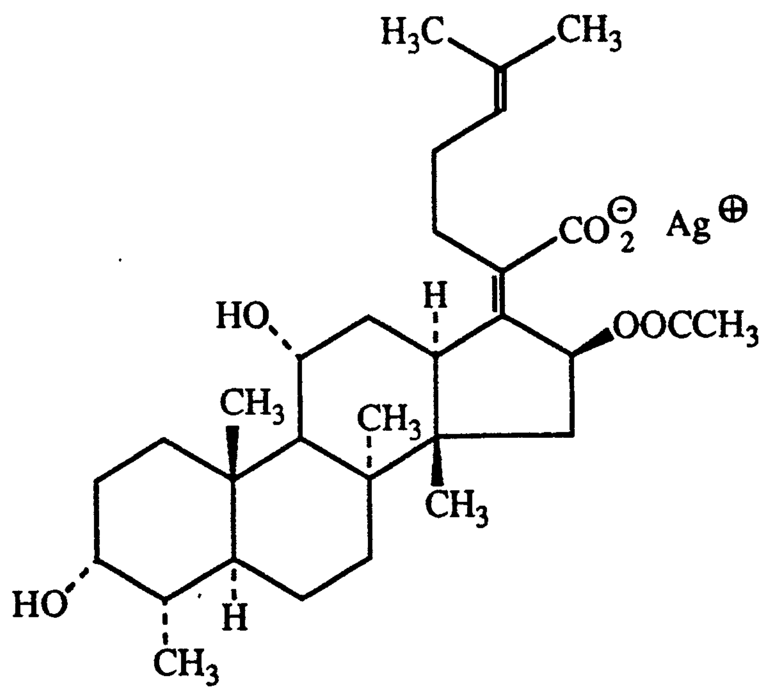

Silver fusidate may be prepared by reacting stoichiometric quantities of sodium fusidate and silver nitrate in a suitable polar or non-polar solvent. As indicated above, silver fusidate is insoluble in water and, accordingly, if the reaction is carried out in an aqueous solution the silver fusidate precipitates as it is

formed and it can be collected following filtration of the medium. The reaction can also be carried out, for example, in ethyl acetate and the silver fusidate precipitated with hexane.

Preparation of silver fusidate from the free acid form using a process analogous to that used for the preparation of known salts such as sodium fusidate should also be feasible.

The invention also provides zinc fusidate which is insoluble in water and hexane and soluble in methanol, ethyl acetate and acetone; melts at 190-193°C; has a molecular weight of 1096.74; has an empirical formula of $C_{62}$ $H_{94}$ Zn $O_{12}$ ; and on thin layer chromatography on silica gel with 85:15 v/v chloroform-methanol with uv visualisation shows one major spot at $R_f$ 0.54.

Zinc fusidate has the structural formula:

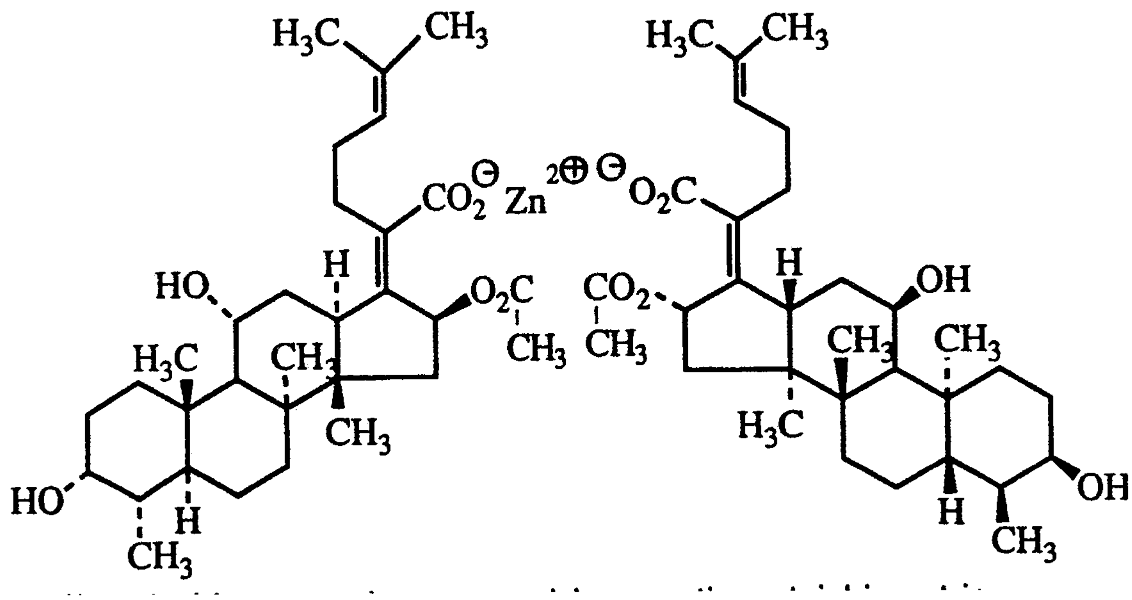

Zinc fusidate may be prepared by reacting stoichiometric quantities of sodium fusidate and either zinc chloride or zinc sulphate in a suitable polar or nonpolar solvent.

Zinc fusidate is also insoluble in water, therefore, if the reaction is carried out in an aqueous solution the zinc fusidate precipitates as it is formed and can be collected by filtration. Alternatively, the reaction can be carried out, for example, in ethyl acetate and the zinc fusidate precipitated with hexane.

Other transition metal salts of fusidic acid can be prepared in like manner.

The invention also provides an anti-infective composition which comprises a transition metal complex or salt of fusidic acid in association with a pharmaceutically acceptable carrier, diluent or excipient therefor.

The anti-infective composition according to the invention is preferably administered topically.

The preferred transition metal salts are silver and zinc as indicated above.

Silver fusidate can be used as an effective anti-infective agent with antibacterial, antifungal and antiviral activity, meeting the requirements of pharmaceutically acceptable antibiotics. As an antibacterial agent it is effective against Gram-positive and Gram-negative bacteria. It is particularly effective in the topical treatment of burns, lesions and open wounds. It can be used for the prevention and treatment of sepsis in the case of second- and third-degree burns, lesions and open wounds. Silver fusidate is particularly effective against species of Clostridium, Proteus, Pseudomonas, Staphylococcus and Streptococcus . In use as an antimicrobial agent silver fusidate is found to be considerably more effective than either silver or fusidic acid alone.

As discussed above for silver fusidate, zinc fusidate can also be used as an effective anti-infective agent with antibacterial, antifungal and antiviral activity. As an antibacterial agent it is especially effective against Gram positive bacteria.

Thus, according to a further aspect of the invention there is provided a method of treatment using a transition metal complex or salt, especially the silver and zinc salts, of fusidic acid as an anti-infective agent

Pharmaceutical compositions in accordance with the invention suitably comprise an anti-infective amount of silver fusidate or zinc fusidate in admixture with a suitable solid or liquid pharmaceutical carrier and optionally appropriate auxilliary agents conventionally used in the formulation of compositions for topical administration. Thus the compositions for topical administration include, for example, creams, dispersions, emulsions, gels, liquids, ointments, powders, solutions, suspensions and dressings impregnated with a solution or suspension of silver fusidate. The compositions may be in unit dosage form and the methods of formulating such dosage forms are well known to those skilled in the art. The compositions may also include an amount of an anti-inflammatory agent such as hydrocortisone or a salt thereof such as hydrocortisone acetate.

The compositions may also be administered orally in the form of tablets or capsules.

The amount of silver or zinc fusidate or other transition metal fusidate administered will depend on the subject being treated, severity of the condition being treated, the mode of administration and the judgement of the prescribing physician.

Formulations for topical administration will suitably contain between 0.1 and 10% by weight of silver or zinc fusidate, more especially 1-5% by weight. Suitably an ointment will contain 2% by weight of silver or zinc fusidate in a white petrolatum or lanolin base. A suitable gel would contain 1% by weight of silver or zinc fusidate. When silver fusidate is used in admixture with an anti-inflammatory agent, the latter would normally be present in an amount which is 50% by weight of the weight of silver fusidate. Similarly when zinc fusidate is used with an anti-inflammatory agent, the amount of anti-inflammatory agent usually present is 50% by weight of the weight of zinc fusidate.

The salts of the present invention can be suitably formulated as ear, eye and nose drops and other formulations suitable for the treatment of infections of the ears, eyes and nose. In the case of ear drops the fusidate salt may be suitably suspended in an animal, mineral or vegetable oil, preferably a vegetable oil such as almond oil, castor oil, coconut oil, cotton seed oil, olive oil, peanut oil, poppy-seed oil, sesame oil or sunflower oil.

The silver or zinc fusidate or other transition metal fusidate salt may be micronised for use in any of the aforementioned formulations.

The invention will be further illustrated by the following Examples.

## EXAMPLE 1

### Preparation of silver salt of fusidic acid

Silver nitrate (32 mg) in water (5 ml) is mixed with sodium fusidate (100 mg) in warm water (5 ml). The resulting precipitate is filtered off, washed with hot water and dried to give silver fusidate (84 mg, 73%) m.p. 200-202°C, TLC on silica gel (Merck 5534), with 85:15 v/v chloroform-methanol and uv visualisation shows one major spot at $R_f$ 0.65 with trace impurities.

| Elemental analysis $C_{31}H_{47}AgO_6$ (mw 623.55) | | |
|---|---|---|
| Element | Theory | Found |
| C | 59.71 | 58.16 |
| H | 7.60 | 7.45 |
| O | 15.40 | 15.81 |
| Ag | 17.30 | 17.23 |

The silver content was determined by atomic absorption.

The silver fusidate thereby prepared is found to be insoluble in water and hexane, but soluble in methanol, ethyl acetate and acetone.

## EXAMPLE 2

### Preparation of silver salt of fusidic acid

Example 1 is repeated except that the reaction is carried out in ethyl acetate and the silver fusidate is precipitated with hexane, followed by filtration and drying

EXAMPLE 3

Preparation of zinc salt of fusidic acid

Zinc sulphate (0.3g) in water (6ml) is mixed with sodium fusidate (1.14g) in water (57ml). The resulting precipitate is filtered off, washed with water and dried to give zinc fusidate (0.91g, 41%). Recrystallization from acetone-hexane gives a material having a melting point of 190° -193° C. TLC on silica gel (Merck 5534), with 85:15 v/v chloroform-methanol and uv visualisation shows one major spot at $R_f$ 0.54.

| Elemental analysis $C_{62}$ $H_{94}$ Zn $O_{12}$ (mw 1096.74) | | |
|---|---|---|
| Element | Theory | Found |
| C | 67.89 | 65.17% |
| H | 8.64 | 8.69% |
| O | 17.51 | 19.05 |
| Zn | 5.96 | 6.70% |

The zinc content was determined by atomic absorption.

The zinc fusidate thereby prepared is found to be insoluble in water and hexane, but soluble in methanol, ethyl acetate and acetone.

EXAMPLE 4

Preparation of zinc salt of fusidic acid

Example 1 is repeated except that the reaction is carried out in ethyl acetate and the zinc fusidate is precipitated with hexane, followed by filtration and drying.

EXAMPLE 5

Preparation of zinc salt of fusidic acid

Example 3 is repeated except that zinc chloride is used in place of zinc sulphate in the starting materials.

Pharmaceutical Preparations

Ointment

An ointment is prepared from the following ingredients.

| Ingredient | Amount |
|---|---|
| Silver fusidate | 10 g. |
| Stearyl alcohol | 40 g. |
| White wax | 80 g. |
| White petrolatum | 870 g. |

The stearyl alcohol, white wax and white petrolatum are melted over a steam bath and allowed to cool. The silver fusidate is added slowly to the ointment base with stirring.

Ointment

An ointment is prepared from the following ingredients:

| Ingredient | Amount |
|---|---|
| Silver fusidate | 10 g. |
| Hydrocortisone acetate | 5 g. |
| Stearyl alcohol | 40 g. |
| White wax | 80 g |
| White petrolatum | 865 g. |

The stearyl alcohol, white wax and white petrolatum are melted together over a steam bath. The silver fusidate and hydrocortisone acetate are then added to the mixture gradually with stirring.

Ointment

An ointment is prepared from the following ingredients.

| Ingredient | Amount |
|---|---|
| Silver fusidate | 2 g. |
| Liquid paraffin | 5 g. |
| White soft paraffin | to 100 g. |

The paraffin components are transferred to a heated container and melted with occasional stirring. The silver fusidate is sieved and as soon as the paraffin base is entirely melted the silver fusidate is added thereto and stirring is continued until the mixture is cold. Once the base solidifies stirring is discontinued to avoid excessive aeration.

Ointment

An ointment is prepared from the following ingredients.

| Ingredient | Amount |
|---|---|
| Zinc fusidate | 5 g. |
| Stearyl alcohol | 20 g. |
| White wax | 40 g. |
| White petrolatum | 435 g. |

The stearyl alcohol, white wax and white petrolatum are melted over a steam bath and allowed to cool. The zinc fusidate is added slowly to the ointment base with stirring.

Ointment

An ointment is prepared from the following ingredients.

| Ingredient | Amount |
|---|---|
| Zinc fusidate | 1 g. |
| Hydrocortisone acetate | 0.5 g. |
| Stearyl alcohol | 4.0 g. |
| White wax | 8.0 g |
| White petrolatum | 86.5 g. |

The stearyl alcohol, white wax and white petrolatum are melted together over a steam bath. The zinc fusidate and hydrocortisone acetate are then added to the mixture gradually with stirring.

| Cream | |
|---|---|
| Ingredient | Amount |
| Silver fusidate | 2 g. |
| Aqueous cream B.P. | 98 g. |

The cream base is warmed to 60°C. Finely powdered silver fusidate is added to the base and stirring is continued until the silver fusidate is evenly distributed in the base. Stirring is continued until the mixture cools. Stirring should not be at too high a speed so as to avoid incorporating air bubbles into the cream.

| Cream | |
|---|---|
| Ingredient | Amount |
| Zinc fusidate | 2 g. |
| Aqueous cream B.P. | 98 g. |

The cream base is warmed to 60°C. Finely powdered zinc fusidate is added to the base and stirring is continued until the zinc fusidate is evenly distributed in the base. Stirring is continued until the mixture cools. Stirring should not be at too high a speed so as to avoid incorporating air bubbles into the cream.

| Gel | |
|---|---|
| Ingredient | Amount |
| Silver fusidate | 3.00 g |
| Carbopol (Carbopol is a Trade Mark) 934 | 2.00 g |
| Triethanolamine | 1.65 ml |
| Methyl hydroxybenzoate | 0.15 g |
| Propyl hydroxybenzoate | 0.05 g |
| Purified water | to 100.00 ml |

The silver fusidate, methyl hydroxybenzoate and propyl hydroxybenzoate are dispersed in 95 ml of freshly boiled and cooled purified water using vigorous mechanical stirring. The Carbopol is added in small amounts while continuing to stir vigorously until a uniform cloudy dispersion is obtained. The mixture is then allowed to stand so that air bubbles may separate, followed by gentle stirring to avoid entrapping air. The triethanolamine is then added a drop at a time and finally the remaining water is added.

| Gel | |
|---|---|
| Ingredient | Amount |
| Zinc fusidate | 1.00 g |
| Carbopol (Carbopol is a Trade Mark) 934 | 0.70 g |
| Triethanolamine | 0.55 ml |
| Methyl hydroxybenzoate | 0.05 g |
| Propyl hydroxybenzoate | 0.02 g |
| Purified water | to 30.00 ml |

The zinc fusidate, methyl hydroxybenzoate and propyl hydroxybenzoate are dispersed in 25 ml of freshly boiled and cooled purified water using vigorous mechanical stirring. The Carbopol is added in small amounts while continuing to stir vigorously until a uniform cloudy dispersion is obtained. The mixture is then allowed to stand so that air bubbles may separate, followed by gentle stirring to avoid entrapping air. The triethanolamine is then added a drop at a time and finally the remaining water is added.

| Lotion | |
|---|---|
| Ingredient | Amount |
| Silver fusidate | 2.00 g |
| Sterile methylcellulose (4,500) solution (2%) | 50.00 ml |
| Benzalkonium chloride | 0.05 g |
| Sterile water | to 100.00 ml |

The benzalkonium chloride is dissolved in about 20 ml of the sterile water. The silver fusidate is dispersed in the methycellulose solution by means of vigorous stirring. The methylcellulose (4,500) used is a high viscosity grade. The solution of benzalkonium chloride is then added slowly while stirring is continued. The lotion is then brought up to the desired volume with the remaining water. Preparation of the lotion is carried out under aseptic conditions.

| Dusting Powder | |
|---|---|
| Ingredient | Amount |
| Silver fusidate | 1 g |
| Sterilised absorbable maize starch B.P. dusting powder | 99 g |

The dusting powder is formulated by gradually adding the sterilized absorbable dusting powder to the silver fusidate to form a uniform blend. The powder is then sterilized in conventional manner.

| Dusting Powder | |
|---|---|
| Ingredient | Amount |
| Zinc fusidate | 1 g |
| Sterilised absorbable maize starch B.P. dusting powder | 99 g |

The dusting powder is formulated by gradually adding the sterilized absorbable dusting powder to the zinc fusidate to form a uniform blend. The powder is then sterilized in conventional manner.

Ear Drops

Ear drops are prepared from the following ingredients:

| Ingredient | Amount |
|---|---|
| Silver fusidate | 15.00 g. |
| Cotton seed oil | to 1000.00 g. |

The silver fusidate is suspended in the cotton seed oil with stirring. In use the ear drops are instilled in the auditory canal of the ear. In general between 2 and 20 drops of the suspension will be instilled in the auditory canal twice daily with gentle massaging of the outer ear.

Ear Drops

Ear drops are prepared from the following ingredients:

| Ingredient | Amount |
|---|---|
| Zinc fusidate | 5.00 g. |
| Cotton seed oil | to 350.00 g. |

The zinc fusidate is suspended in the cotton seed oil with stirring. In use the ear drops are instilled in the auditory canal of the ear. In general between 2 and 20 drops of the suspension will be instilled in the auditory canal twice daily with gentle massaging of the outer ear.

Ear Drops

Ear drops are prepared from the following ingredients:

| Ingredient | Amount |
|---|---|
| Silver fusidate | 0.10 g |
| Polysorbate 20 | 0.10 g |
| Purified water | to 10.00 ml |

The polysorbate is dissolved in approximately 9 ml of the purified water and then the silver fusidate is added gradually with thorough mixing. The volume is then adjusted to 10 ml with the remaining purified water.

Nasal Drops

Nasal drops are prepared from the following ingredients:

| Ingredient | Amount |
|---|---|
| Silver fusidate | 0.10 g |
| Polysorbate 20 | 0.10 g |
| Phosphate buffer | to 10.00 ml |

The polysorbate is dissolved in approximately 9 ml of the phosphate buffer and then the silver fusidate is added gradually with thorough mixing. The volume is then adjusted to 10 ml with the remaining phosphate buffer.

The phosphate buffer solution is buffered to pH 6.5 and is isoosmotic. The phosphate buffer has the following composition:

| Ingredient | Amount |
|---|---|
| Sodium acid phosphate solution | 70.00 ml |
| Sodium phosphate solution | 30.00 ml |
| Sodium chloride | 0.50 g |

Nasal Drops

Nasal drops are prepared from the following ingredients:

| Ingredient | Amount |
|---|---|
| Zinc fusidate | 0.10 g |
| Polysorbate 20 | 0.10 g |
| Phosphate buffer | to 10.00 ml |

The polysorbate is dissolved in approximately 9 ml of the phosphate buffer and then the silver fusidate is added gradually with thorough mixing. The volume is then adjusted to 10 ml with the remaining phosphate buffer.

The phosphate buffer solution is buffered to pH 6.5 and is isoosmotic. The phosphate buffer has the following composition:

| Ingredient | Amount |
|---|---|
| Sodium acid phosphate solution | 70.00 ml |
| Sodium phosphate solution | 30.00 ml |
| Sodium chloride | 0.50 g |

Eye Drops

Eye drops are prepared from the following ingredients:

| Ingredient | Amount |
|---|---|
| Silver fusidate | 0.10 g |
| Polysorbate 20 | 0.10 g |
| Phenylmercuric nitrate | 2.00 mg |
| Phosphate-citrate buffer (pH7.5) (using sterile water) | to 10.00 ml |

The phenylmercuric nitrate is dissolved in approximately 9 ml of the buffer solution, with gentle heating if required. The polysorbate 20 is then added and allowed to dissolve. The silver fusidate is then added gradually and the volume adjusted to 10 ml with the remaining buffer solution. The final product is clarified and sterilised in conventional manner.

Eye Drops

Eye drops are prepared from the following ingredients:

| Ingredient | Amount |
|---|---|
| Zinc fusidate | 0.10 g |
| Polysorbate 20 | 0.10 g |
| Phenylmercuric nitrate | 2.00 mg |
| Phosphate-citrate buffer (pH7.5) (using sterile water) | to 10.00 ml |

The phenylmercuric nitrate is dissolved in approximately 9 ml of the buffer solution, with gentle heating if required. The polysorbate 20 is then added and allowed to dissolve. The zinc fusidate is then added gradually and the volume adjusted to 10 ml with the remaining buffer solution. The final product is clarified and sterilised in conventional manner

## IN VITRO ASSESSMENT OF SILVER FUSIDATE

Minimum inhibitory concentrations (MIC), minimum bactericidal concentrations (MBC), rates of killing and the incidence of naturally occurring resistant mutants were determined for silver fusidate prepared according to Example 1. MICs were determined by the plate dilution method using IsoSensitest (IsoSensitest is a Trade Mark) media supplemented where necessary with 5% lysed horse blood. Two inocula were used, $10^6$ and $10^4$ cfu. MICs were read after overnight incubation in the appropriate atmosphere. The plates bearing the $10^6$ inocula were replicated using velvet pads for the determination of MBC which is defined as the lowest antibiotic concentration killing at least 99.9% of the original inoculum.

20 strains of the following species were tested:

Gram positive: methicillin-resistant Staph. aureus (MRSA), methicillin-sensitive Staph. aureus (MSSA),

methicillin-resistant Staph. epidermidis (MRSE), Enterocci , Streptococcus pyogenes , Candida albicans , Clostridium spp .

Gram negative: Escherichia coli , Enterobacter spp. , Klebsiella pneumoniae , Acinetobacter spp. , Pseudomonas aeruginosa , Providencia stuartii , Proteus mirabilis , indole-positive Proteae (10 each of Prov. rettgeri and Pr. vulgaris ).

The data indicate that silver fusidate has a broad spectrum of activity against Gram positive and Gram negative bacteria and Candida albicans , especially when compared to the activity of sodium fusidate and silver sulphadiazine. The activity of silver fusidate against Gram negative bacteria is particularly significant when one considers the total lack of Gram negative activity observed with sodium fusidate.

For example, with regard to Gram positive activity the MIC range (µg/ml) observed for silver fusidate when tested against MRSA at an inoculum of $10^6$ org/ml was 0.13-64 and the observed MBC range (µg/ml) was 32->128. This compares favourably with the 0.5->128 MIC range and 16->128 MBC range observed for sodium fusidate and the 32->128 range and 128-256 MBC range observed for silver sulphadiazine. With regard to the Gram negative activity, the observed MIC and MBC ranges for silver tusidate when tested against, for example, Pseudomas sp. , were each 32-64 compared with a MIC range of 1024->2048 and MBC range of 2048->2048 observed with sodium fusidate. The remaining Gram negative organisms when tested demonstrated similar comparative differences in their respective MIC and MBC ranges.

During the course of the in vitro assessment of silver fusidate it was observed that one particular strain of Staphyloccus was killed more rapidly in a non-growing state. This is a very interesting observation because it is known that certain strains of Staphylococcus colonize, e.g., the nose, creating in the host a carrier state. Carrier state hosts are a constant source of potential infection both for themselves and those they come in contact with. This is a particularly troublesome source of infections in the hospital environment. It is recognized that the cheapest and most efficient way off removing this problem from a hospital is to remove or discharge the carrier. This, of course, is not always possible, practical or even ethical, therefore, any anti-infective compound effective in killing non-growing state organisms has significant potential in eradicating colonized bacteria from carriers.

It was also observed during the in vitro assessment of silver fusidate against various strains of Staphylococcus that the incidence of silver fusidate resistant mutants was too low to be measured, as evidenced by the absence of growth in the presence of 100 µg/ml of silver tusidate. This suggests that resistance development may not be a problem especially under the conditions for which a topical agent containing, i.e. a cream or ointment having 1% silver fusidate, would be used.

## IN VITRO ASSESSMENT OF ZINC FUSIDATE

MICs were determined for zinc fusidate prepared according to Example 3 using the following Gram positive and Gram negative panels:

4 strains of Staphylococcus aureus (2 methicillin-resistant, 2 methicilllin-sensitive) and 2 strains each of coagulase-negative staphylococci , Staph. saprophyticus , Enterococci , Streptococcus pyogenes , Strept . agalactiae , Listeria monocytogenes , diphtheroids and Bacillus spp. , 2 strains each of Escherichia coli , Klebsiella pneumoniae , Pseudomonas aeruginosa , Providencia stuartii , Proteus morganii , Salmonella typhimurium , Acinetobacter spp. , Citrobacter freundii, Serratia marcescens and Enterobacter cloacae .

The data indicate that zinc fusidate demonstrates activity against Gram positive bacteria. For example, a MIC (µg/ml) range of 10-100 was observed for coagulase negative Staphylococci and an MIC range of <1-10 was observed for the remaining Gram positive organisms.

## Claims

1. A transition metal complex or salt of fusidic acid.
2. A transition metal complex or salt of fusidic acid according to Claim 1, characterised in that the transition metal is silver or zinc.
3. Silver fusidate.
4. Zinc fusidate.
5. A transition metal complex or salt of fusidic acid according to any preceding claim for use as an anti-infective agent.
6. An anti-infective composition, characterised in that it comprises a transition metal complex or salt of

fusidic acid according to any one of Claims 1-4 in association with a pharmaceutically acceptable carrier, diluent or excipient therefor.

7. A composition according to Claim 6, characterised in that it is suitable for topical administration.

8. A composition according to Claim 6 or 7, characterised in that it includes an amount of an anti-inflammatory agent.

9. A composition according to any one of Claims 6-8, characterised in that it is in the form of a cream, dispersion, emulsion, gel, liquid, ointment, powder, solution or suspension.

10. A dressing for topical application characterised in that it is impregnated with a composition according any one of Claims 7-9.

11. A process for the preparation of a transition metal complex or salt of fusidic acid as defined in any one of claims 1 to 4 which comprises reacting fusidic acid or an alkali metal, alkaline earth metal, ammonium or amine salt thereof with a transition metal salt.